# EUROPEAN PATENT APPLICATION

(11) **EP 3 156 011 A1**
(43) Date of publication of application: **19.04.2017**
(21) Application number: 15807438.5
(22) Date of filing: 11.06.2015
(51) Int. Cl.: A61F 2/82, A61F 2/07

(54) **STENT**

(30) Priority: 12.06.2014 JP 2014121080
(71) Applicant: National Cerebral and Cardiovascular Center, Suita, Osaka 565-8565 (JP); The School Corporation Kansai University, Suita-shi, Osaka 564-8680 (JP)
(72) Inventor: NAKAYAMA, Yasuhide, Suita-shi Osaka 565-8565 (JP); SATO, Tetsu, Suita-shi Osaka 565-8565 (JP); MORIWAKI, Takeshi, Suita-shi Osaka 565-8565 (JP); TAJIKAWA, Tsutomu, Suita-shi Osaka 564-8680 (JP)
(74) Representative: Müller Hoffmann & Partner
(86) International application number: PCT/JP2015/066805
(87) International publication number: WO 2015/190541

(57) **Abstract**

The present invention provides a stent that can obtain sufficient strength while an inner side of the stent is easily endothelialized and moreover, can prevent occlusion of a branch blood vessel. Specifically, a stent body capable of diameter expansion is provided. A tubular film 3 is held by the stent body. In the tubular film 3, a plurality of communication holes 4 communicating between an inside and an outside are formed. Band-shaped portions 7 and 8 are formed between the communication holes 4 adjacent to each other. A wide-width portion and a narrow portion are not generated in the band-shaped portions 7 and 8. Strength of a part of the band-shaped portions 7 and 8 is not lowered. Generation of the wide-width portion in the band-shaped portions 7 and 8 is prevented.

## Description

### Technical Field

The present invention relates to a stent including a stent body capable of diameter expansion and a tubular film held by the stent body.

### Background Art

In the case of constriction in a lumen such as a blood vessel, treatment is carried out by conveying a cylindrical implant in the lumen called a stent to a constriction portion through an inside of the blood vessel or the like and by expanding a diameter of the stent so as to open this constriction portion wide from the inside and by supporting it in some cases. Moreover, other than a constriction-portion expansion technique for treating the constriction portion, use of the stent in various operations such as an aneurysm occlusion technique in which the stent is implanted in a portion where the aneurysm is generated and treatment is carried out by occluding the aneurysm with respect to the blood vessel has been examined.

Patent Literature 1, for example, discloses a stent which prevents metal allergy and the like by covering an entire surface of the stent body having a tubular shape capable of diameter expansion and made of metal with a soft polymer film and suppresses occurrence of a thrombus caused by a disturbance in a blood flow by smoothening an inner surface of the stent. Moreover, the stent in Patent Literature 1 allows invasion of endothelial cells from micro pores so as to promote endothelialization of the inner side of the stent by forming a plurality of the micro pores on the polymer film and suppresses occurrence of a thrombus or thickening of an inner membrane caused by implantation of the stent which is a foreign substance in the blood vessel.

As in Patent Literature 1, in the stent in which a pore is formed in the film covering the stent body, by setting its opening rate (pore area per unit area of the film) to an appropriate value, the film in a periphery of the pore can be utilized as a base for promoting endothelialization of an inner side of the stent while invasion of endothelial cells into the inner side of the stent is facilitated.

Moreover, when the stent is to be used for treatment of aneurysm, by setting its opening rate to an appropriate value, a blood which is to flow into the aneurysm from the blood vessel can be sufficiently shut down and moreover, even if the blood vessel branches from a portion where the stent is to be implanted, the branch blood vessel can be prevented from being completely blocked by the stent.

### Citation List

### Patent Literature

Patent Literature 1: Japanese Patent Laid-Open No. 2004-261567 (claims 1, 2, 5, paragraphs 0012, 0017, 0024 to 0027)

### Summary of Invention

### Technical Problem

However, even if pores are formed in a film of a stent at a predetermined opening rate, when the pores are formed only at constant central intervals, a wide portion and a narrow portion are generated easily in the film between the pores. In this case, in a portion away from a pore peripheral edge in the wide-width portion of the film, there is a concern that endothelialization of the inner side of the stent is delayed since the endothelial cells such as a blood vessel cannot invade easily, and moreover, when the stent is implanted in the blood vessel, there is a concern that the film of the stent blocks the branch blood vessel since the wide-width portion of the film between the pores is located at a branch part of the blood vessel.

In order to solve these problems, elimination of the wide-width portion of the film between the pores by narrowing the center interval of the pores formed in the film can be considered, but since the film between the pores generally becomes narrow, there is a concern that the narrow-width portion of the film becomes narrower and strength of the film is deteriorated.

The present invention has an object to provide a stent which can obtain sufficient strength while the inner side of the stent is easily endothelialized and moreover, occlusion of a branch blood vessel can be prevented.

### Solution to Problem

In order to achieve the aforementioned object, a stent according to the present invention includes a stent body capable of diameter expansion and a tubular film held by the stent body, in which a plurality of communication holes communicating between an inside and an outside is formed in the tubular film, and a band-shaped portion having a constant width in a diameter-expanded state of the stent body is formed between the communication holes adjacent to each other.

According to the aforementioned constitution, since the band-shaped portion between the communication holes in the tubular film is formed having the constant width in the diameter-expanded state, a wide-width portion and a narrow portion are not generated, whereby strength of a part of the band-shaped portion is not lowered, and a width of the band-shaped portion is generally suppressed, and generation of the wide-width portion can be prevented. As a result, portions away from the pore peripheral edge are reduced, the endothelial cells can be easily made to invade into the substantially entire inner side of the stent for endothelialization and moreover, occlusion of a branch blood vessel caused by the wide-width portion can be prevented.

Here, the aforementioned stent only needs to be such that the band-shaped portion of the tubular film has the constant width in the diameter-expanded state of the stent body, and the stent body may be forcedly expanded by a balloon or may be formed of a shape memory alloy so that the stent body expands itself. Moreover, the aforementioned stent can be used for various lumen portions of a human body such as a cerebral artery, a coronary artery, a bile duct, an esophageal, an air tube, a prostate, a ureter, an oviduct, an aorta, a peripheral artery, a renal artery, a carotid artery, a cerebral blood vessel and the like, and particularly by using an extremely thin stent, it can be used for treatment in a cerebral surgery field.

Moreover, the stent may be such that the communication holes are set to have a hexagonal shape in the diameter-expanded state of the stent body.

According to this constitution, since the communication holes are set having a hexagonal shape, a film in a periphery of the communication hole can be constituted with a honeycomb structure, and sufficient strength can be obtained while the opening rate of the tubular film is improved to a desired value.

Moreover, the stent may also be such that the communication holes are set to have a diamond shape in the diameter-expanded state of the stent body.

According to this constitution, since the communication holes are set having a diamond shape, when a diameter of the stent body is to be expanded, a peripheral edge portion of the communication holes can be made to follow deformation of the stent body while it is deformed like a pantograph. The communication holes only need to be such that the band-shaped portion with a constant width can be constituted between the communication holes and can be any shape including a square, a triangle, an arc and the like other than the diamond, not limited only to a hexagon or a diamond.

Moreover, the present invention provides a stent including a stent body capable of diameter expansion and a tubular film held by the stent body, in which a plurality of communication holes communicating between an inside and an outside is aligned in a stent circumferential direction and in plural rows in the tubular film, the communication holes in the plural rows are formed with positions in the stent circumferential direction alternately shifted, and the communication holes in the rows adjacent to each other are formed with end portions in a stent center axis direction overlapped in the stent circumferential direction.

According to the aforementioned constitution, since the end portions of the communication holes in the rows adjacent to each other are overlapped in the stent circumferential direction, by expanding the diameter of the stent body and by forcedly stretching the tubular film in the stent circumferential direction, a force for pulling in the stent circumferential direction can be applied only to the center part excluding the end portions in the peripheral edge portion of the communication holes. As a result, a portion in the peripheral edge of the communication holes crossing the stent circumferential direction can be deformed into a mountain shape while the communication holes are expanding in the stent circumferential direction, whereby the peripheral edges of the communication holes adjacent to each other can be deformed substantially in parallel, and the band-shaped portion between the communication holes can be formed having a constant width.

It is only necessary that the communication holes have their peripheral edge deformed into a mountain shape when the tubular film is stretched in the stent circumferential direction, and thus, the phrase that "the plurality of communication holes is aligned in the stent circumferential direction and in plural rows" does not necessarily mean that the communication holes are accurately aligned in the stent circumferential direction but also includes a concept that the communication holes are aligned with inclination to the stent circumferential direction.

The communication holes can be formed having various shapes, and they can be set having a rectangular shape in which a pair of opposite sides is in parallel with the stent center axis direction in the state before diameter expansion of the stent body, for example.

According to this constitution, since the communication holes before the diameter expansion are made a rectangle, and their pair of opposite sides is set in parallel with the stent center axis direction, by expanding the diameter of the stent body, the pair of opposite sides are deformed into the mountain shapes and the rectangular communication holes can be deformed into a hexagon, whereby the tubular film after the diameter expansion can be constituted with a honeycomb structure. Moreover, by setting lengths of sides (remaining opposite sides) sufficiently shorter than the pair of opposite sides of the rectangle, the communication holes after the diameter expansion can be constituted having a diamond shape.

Moreover, the communication holes may be set having a hexagonal shape in which a pair of opposite sides is in parallel with the stent center axis direction in the state before the diameter expansion of the stent body.

According to this constitution, since the communication holes before the diameter expansion are made a rectangle, and their pair of opposite sides are set in parallel with the stent center axis direction, by expanding the diameter of the stent body, the pair of opposite sides can be deformed into mountain shapes, and the hexagonal communication holes can be deformed into a diamond shape.

Moreover, the communication holes may be set to have an elliptic shape in which a long axis is in parallel with the stent center axis direction in the state before the diameter expansion of the stent body.

According to this constitution, since the communication holes before the diameter expansion are set to have an elliptic shape with the long axis in parallel with the stent center axis direction, by expanding the diameter of the stent body, a pair of portions sandwiching the long axis in the peripheral edge of the communication holes can be deformed into mountain shapes, and the elliptic communication hole can be deformed into a diamond shape.

The plurality of communication holes do not have to have the same shape for all but a combination of the communication holes with different shapes such as a rectangle and a regular square or a combination of the communication holes with different sizes and the like can be employed.

### Advantageous Effect of Invention

As described above, according to the present invention, the plurality of communication holes are formed in the tubular film held by the stent body, and the band-shaped portion between the communication holes adjacent to each other is set to have a constant width in the diameter-expanded state. As a result, since generation of a wide-width portion in the band-shaped portion can be prevented, the endothelial cells can be made to invade into substantially the entire inner side of the stent so as to be endothelialized easily and sufficient strength can be obtained by preventing lowering of the strength in a part of the band-shaped portion and moreover, occlusion of the branch blood vessel by the wide-width portion can be prevented.

### Brief Description of Drawings

[Figure 1] Figures 1 are perspective views of a stent according to the present invention, in which Figure 1(a) illustrates a state before diameter expansion, and Figure 1(b) illustrates a state after the diameter expansion.
[Figure 2] Figure 2 is a sectional view of essential parts of a stent body and a tubular film.
[Figure 3] Figure 3 is an enlarged view of an essential part of the tubular film before the diameter expansion of a stent.
[Figure 4] Figures 4 are views illustrating deformation of rectangular communication holes by the diameter expansion of the stent.
[Figure 5] Figures 5 illustrate shapes after the diameter expansion of the stent of other rectangular communication holes and views for explaining differences in dimensions and arrangement of the communication holes before the diameter expansion of the stent.
[Figure 6] Figures 6 are views illustrating shapes after the diameter expansion of the stent of a hexagonal communication holes.
[Figure 7] Figure 7 are views illustrating shapes after the diameter expansion of the stent of elliptic communication holes.

### Description of Embodiment

Embodiments of a stent according to the present invention will be described below by using the attached drawings.

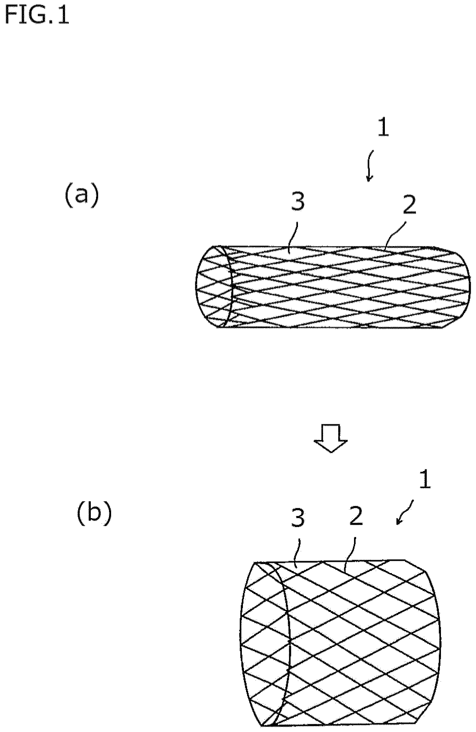

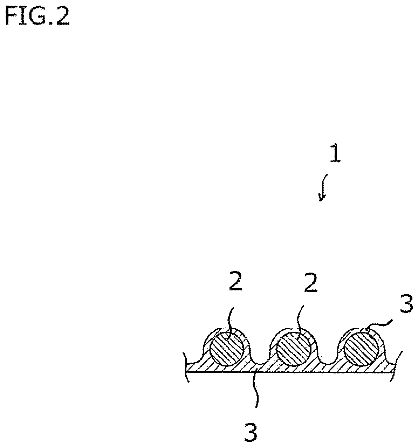

As illustrated in Figures 1 to 3, a stent 1 is for treatment by widening from inside a constriction portion generated within a lumen such as a blood vessel or for treatment by occluding an aneurysm with respect to the blood vessel and includes a stent body 2 capable of diameter expansion and a tubular film 3 held by the stent body 2, in which a plurality of communication holes 4 communicating between an inside and an outside is aligned in a stent circumferential direction and in a plural rows and moreover, the communication holes 4 in the plural rows are formed with stent circumferential positions alternately shifted, and the communication holes 4 in the rows adjacent to each other are formed with end portions in a stent center axis direction overlapped in the stent circumferential direction.

The stent body 2 has a mesh-state structure made of metal forming a tubular shape in general and capable of flexible diameter expansion and is set with a length to approximately 10 to 100 mm, a diameter to approximately 1/10 to 1/2 of the length and a thickness to approximately 30 to 500 µm before the diameter expansion, and the diameter is expanded to a diameter of approximately twice of that before the expansion. Metal constituting the stent body 2 includes stainless steel, titanium, tantalum, aluminum, tungsten, nickel-titanium alloy, cobalt-chromium-nickel-iron alloy and the like, for example, with biocompatibility. This stent body 2 may have self-expandability by memorizing a shape through application of thermal treatment or the diameter may be expanded forcedly by a balloon.

The tubular film 3 is made of polymer elastomer with high flexibility, for example, has a film thickness and a thickness of a covered portion of 5 to 100 µm and covers the entire surface of the stent body 2. The polymer elastomer includes segmented polyurethane, polyolefin-based polymer, and silicone-based polymer, for example, and particularly segmented polyurethane polymer, which is suitable because it is excellent in antithrombogenicity and is also excellent in characteristics such as strength, extensibility and the like, and capable of following the diameter expansion of the stent 1 without fracture. The tubular film 3 does not necessarily have to cover the entire surface of the stent body 2 but may only be wound simply around the stent body 2, for example.

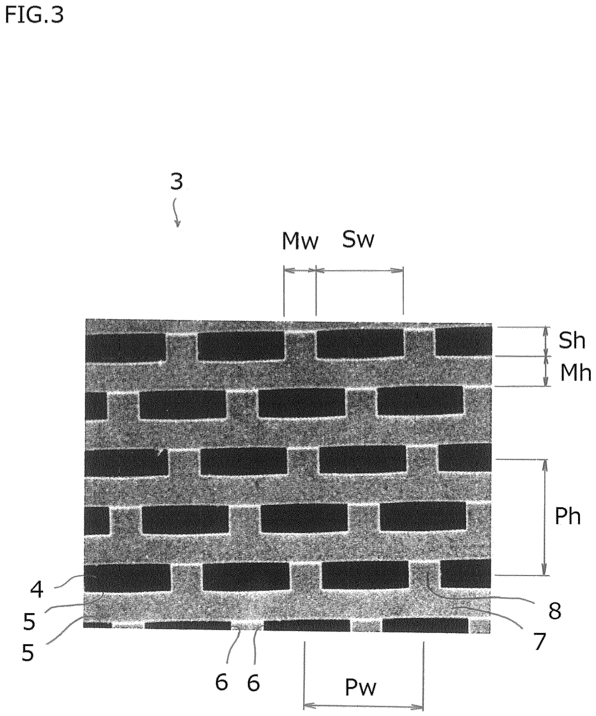

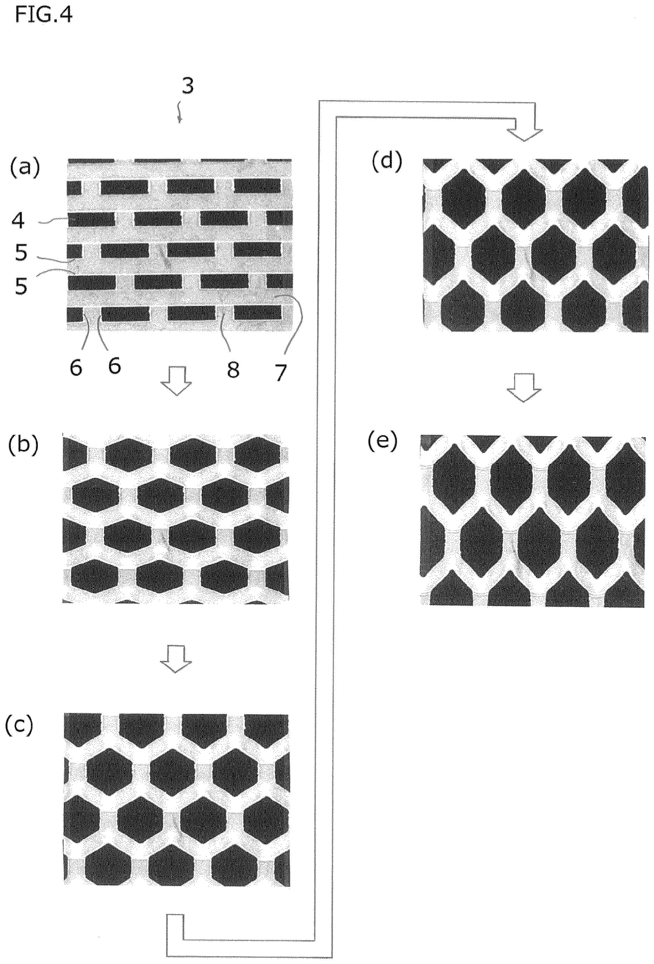

As illustrated in Figures 3 and 4, a communication hole 4 is set to a laterally long rectangle in which a pair of opposite sides 5 is in parallel with a stent center axis direction (right and left direction in Figure 3) and a pair of sides 6 is in parallel with a stent circumferential direction (vertical direction in Figure 3) in a state before diameter expansion of the stent body 2.

Here, by using reference characters described in Figure 3, a shape and arrangement of the communication hole 4 before diameter expansion of the stent will be described. Assuming that a length of the side 6 as a height (Sh) of the communication hole 4 in the stent circumferential direction is 1.0, the other dimensions are indicated in such a ratio that a length of the opposite side 5 as a width (Sw) of the communication hole 4 in the stent center axis direction is 3.3, a pitch (Ph) of the communication hole 4 in the stent circumferential direction is 4.3, a pitch (Pw) of the communication hole 4 in the stent center axis direction is 4.5, a width (Mh) of a vertical band-shaped portion 7 of the communication hole 4 is 1.2, and a width (Mw) of a right-and-left band-shaped portion 8 of the communication hole 4 is 1.2. The height (Sh) of the communication hole 4 in the stent circumferential direction is approximately 30 µm, for example.

By mounting the stent 1 on a balloon catheter and by applying a water pressure, the balloon is expanded, and the diameter of the stent 1 is expanded to approximately 3 mm to 5 mm and then, as illustrated in Figures 4(a) to 4(c), the side 6 is kept as it is, while the pair of opposite sides 5 are deformed into mountain shapes, and the shape of the communication hole 4 is deformed from a laterally long rectangle into a regular hexagon. The widths of the band-shaped portions 7 and 8 in the periphery of the communication hole 4 at this time are substantially a constant width in all the directions, which is approximately 30 µm.

The width (Sw) of the communication hole 4 is 3.3 times of the height (Sh), but since the right-and-left band-shaped portion 8 of the communication hole 4 is stretched in the stent circumferential direction with the diameter expansion, the opposite side 5 deformed into a mountain shape becomes a length approximately twice of the side 6, and the communication hole 4 after the diameter expansion constitutes a regular hexagon.

As illustrated in Figures 4(d) and 4(e), by further expanding the diameter of the stent 1, the communication hole 4 having a regular hexagon is deformed into a vertically long hexagon, but widths of the band-shaped portions 7 and 8 in the periphery thereof maintain a substantially constant width in all the directions.

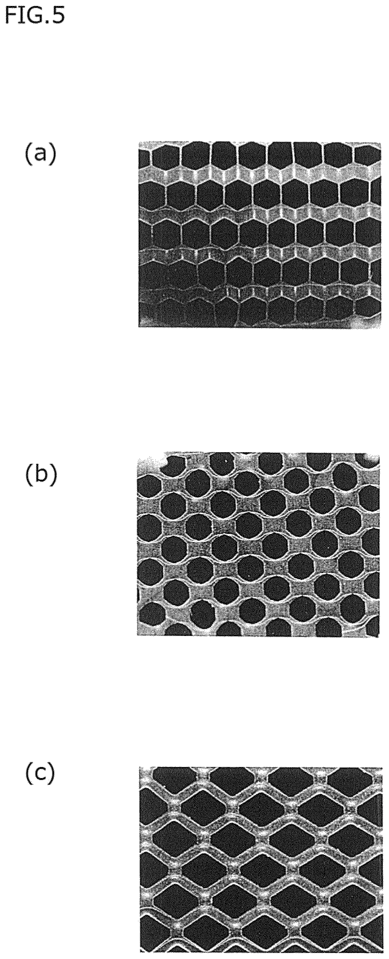

Moreover, as illustrated in Figure 5, by setting the dimensions of the shape and the arrangement of the communication hole 4 before the diameter expansion of the stent to different sizes, the communication hole 4 and the band-shaped portions 7 and 8 in the periphery thereof after the diameter expansion can be set to other shapes.

In Figure 5(a), Sh is set to 1.0, Sw is set to 3.7, Ph is set to 6.7, Pw is set to 4.0, Mh is set to 2.3, and Mw is set to 0.3. In Figure 5(b), Sh is set to 1.0, Sw is set to 3.7, Ph is set to 4.0, Pw is set to 6.7, Mh is set to 1.0, and Mw is set to 3.0. In Figure 5(c), Sh is set to 1.0, Sw is set to 7.0, Ph is set to 5.6, Pw is set to 8.8, Mh is set to 1.8, and Mw is set to 1.8.

As described above, it is known that, before the diameter expansion, the width (Mw) of the right-and-left band-shaped portion 8 of the communication hole 4 is smaller than the width (Sw) of the communication hole 4 in the stent center axis direction, and since end portions of the communication holes 4 are overlapped with each other in the stent circumferential direction, the communication hole 4 can be deformed into a hexagonal shape after the diameter expansion.

Moreover, it is known that before the diameter expansion, by setting the width (Mh) of the vertical band-shaped portion 7 of the communication hole 4 and the width (Mw) of the right-and-left band-shaped portion 8 of the communication hole 4 to 0.5 < Mw/Mh < 2.0, for example, which is substantially the same, the widths of the band-shaped portions 7 and 8 can be made substantially the constant width even after the diameter expansion.

Moreover, before the diameter expansion, it is known that the larger the width (Sw) in the stent center axis direction is set to the height (Sh) of the communication hole 4 in the stent circumferential direction, the closer the shape of the communication hole 4 after the diameter expansion gets to a diamond shape. For example, if it is Sw/Sh > 10, the shape of the communication hole 4 after the diameter expansion can be considered to be a substantially diamond shape.

Subsequently, a method of manufacturing the stent 1 will be described. First, a cylindrical mandrel made of stainless, for example, is immersed in a polyurethane solution so that an outer surface of the mandrel is coated with polyurethane. Then, the stent body 2 having a diameter of 3 mm is strongly overlapped into close contact with an outer side of the coated polyurethane and then, it is immersed in the polyurethane solution so as to be covered therewith, the both surfaces of the stent body 2 are coated with the polyurethane, and a polyurethane film integrated with the stent body 2 and having a combined thickness of approximately 20 µm is formed. The polyurethane solution is 12 weight% solution of segmented polyurethane (Miractran (registered trademark) by Nippon Miractran Co., Ltd., for example) to tetrahydrofuran, for example.

Moreover, after laser machining, the polyurethane film protruding from the both ends of the stent body 2 is cut off and the stent body is immersed in ethanol, and the stent body 2, and the polyurethane film integrated therewith is withdrawn from the mandrel. After that, the communication holes 4 each having a laterally long rectangular shape are formed, by using excimer laser or the like, in plural rows in the polyurethane film integrated with the stent body 2 so as to constitute the tubular film 3, whereby manufacture of the stent 1 is completed.

According to the aforementioned constitution, since the band-shaped portions 7 and 8 having the constant widths are formed in the periphery of the communication hole 4 in the diameter-expanded state, generation of the wide-width portion can be prevented without lowering strength of the tubular film 3, and by making the endothelial cells invade easily into substantially the entire inner side of the stent, endothelialization of the inner side of the stent is promoted, and occlusion of the branch blood vessel by the wide-width portion of the tubular film 3 can be prevented. Moreover, since the diameter of the stent 1 is expanded, and the communication hole 4 is deformed from a laterally long rectangle into a hexagon, formation of the communication hole 4 can be facilitated.

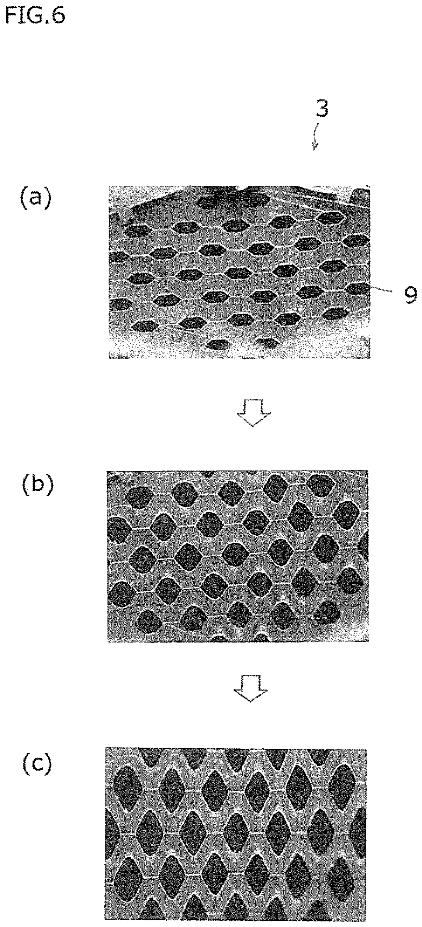

The present invention is not limited to the aforementioned embodiment but can be changed as appropriate within a range of the present invention. For example, as illustrated in Figures 6, in the tubular film 3 before the diameter expansion, a communication hole 9 set having a hexagonal shape in which the pair of opposite sides is in parallel with the stent center axis direction in the state before the diameter expansion of the stent body 2 may be formed instead of the laterally long rectangular communication hole 4. By making the communication hole 9 before the diameter expansion a hexagon (Figure 6(a)), the pair of opposite sides is deformed into mountain shapes while the diameter of the stent body 2 is expanded (Figure 6(b)), and the shape of the communication hole 9 after the diameter expansion is made a diamond shape, whereby the band-shaped portion having the constant width can be formed in the periphery of the communication hole 9 (Figure 6(c)).

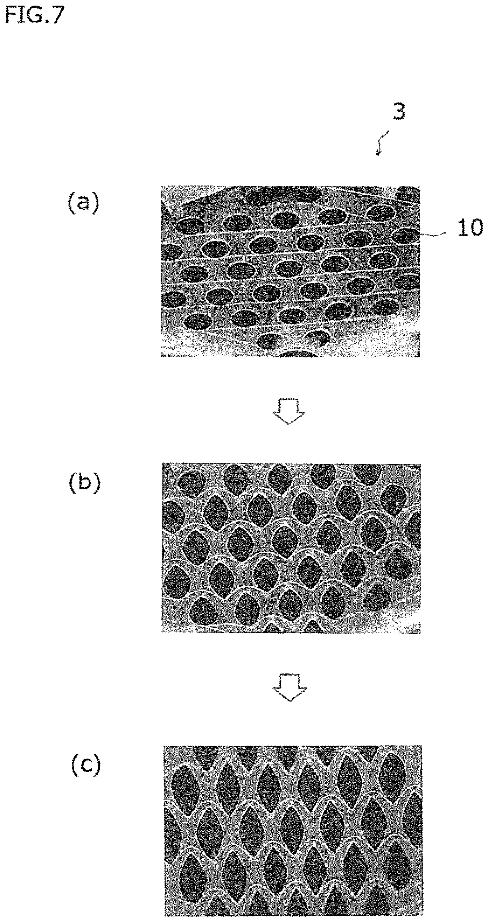

Moreover, as illustrated in Figures 7, an elliptic communication hole 10 with a long axis in parallel with the stent center axis direction in the state before the diameter expansion of the stent body 2 may be formed in the tubular film 3 before the diameter expansion. By making the communication hole 10 before the diameter expansion a laterally long elliptic shape (Figure 7(a)), the both side portions sandwiching the long axis is deformed into mountain shapes while the diameter of the stent body 2 is expanded (Figure 7(b), the shape of the communication hole 10 after the diameter expansion is made a diamond shape, whereby the band-shaped portion having the constant width can be formed in the periphery of the communication hole 10 (Figure 7(c)).

Moreover, it is only necessary that a band-shaped portion having a constant width is formed between the communication holes after the diameter expansion, and the shape of the communication hole before the diameter expansion is not particularly limited.

### Reference Signs List

- 1: stent
- 2: stent body
- 3: tubular film
- 4: communication hole
- 5: opposite side
- 6: side
- 7: band-shaped portion
- 8: band-shaped portion
- 9: communication hole
- 10: communication hole

## Claims

1. A stent comprising a stent body capable of diameter expansion and a tubular film held by the stent body, wherein a plurality of communication holes communicating between an inside and an outside is formed in the tubular film, and a band-shaped portion having a constant width in a diameter-expanded state of the stent body is formed between the communication holes adjacent to each other.

2. The stent according to claim 1, wherein
the communication holes are set to have a hexagonal shape in the diameter-expanded state of the stent body.

3. The stent according to claim 1, wherein
the communication holes are set to have a diamond shape in the diameter-expanded state of the stent body.

4. A stent comprising a stent body capable of diameter expansion and a tubular film held by the stent body, wherein a plurality of communication holes communicating between an inside and an outside is aligned in a stent circumferential direction and in plural rows in the tubular film, the communication holes in the plural rows are formed with stent circumferential positions alternately shifted, and the communication holes in the rows adjacent to each other are formed with end portions in a stent center axis direction overlapped with each other in the stent circumferential direction.

5. The stent according to claim 4, wherein
the communication holes are set to have a rectangular shape in which a pair of opposite sides is in parallel with the stent center axis direction in the state before diameter expansion of the stent body.

6. The stent according to claim 4, wherein
the communication holes are set to have a hexagonal shape in which a pair of opposite sides is in parallel with the stent center axis direction in the state before the diameter expansion of the stent body.

7. The stent according to claim 4, wherein
the communication holes are set to have an elliptic shape in which a long axis is in parallel with the stent center axis direction in the state before the diameter expansion of the stent body.
